# EUROPEAN PATENT APPLICATION

(11) **EP 4 483 777 A1**
(43) Date of publication of application: **01.01.2025**
(21) Application number: 23766508.8
(22) Date of filing: 17.02.2023
(51) Int. Cl.: A61B 1/00, A61B 1/12

(54) **COVER FOR RIGID ENDOSCOPE AND ENDOSCOPE UNIT**

(30) Priority: 11.03.2022 JP 2022038148
(71) Applicant: NAGASAKI UNIVERSITY, Nagasaki 852-8521 (JP); Trytec Co., Ltd., Oita-shi, Oita 870-0278 (JP)
(72) Inventor: MATSUMOTO, Keitaro, Nagasaki-shi, Nagasaki 852-8521 (JP); NAGAYASU, Takeshi, Nagasaki-shi, Nagasaki 852-8521 (JP); TAKAGI, Katsunori, Nagasaki-shi, Nagasaki 852-8521 (JP); TAKEZAKI, Hiroshi, Oita-shi, Oita 870-0278 (JP)
(74) Representative: Page White Farrer
(86) International application number: PCT/JP2023/005828
(87) International publication number: WO 2023/171329

(57) **Abstract**

A cover (2) has a flexible cover member (10) which covers a portion in a circumferential direction of an outer circumferential surface of a rigid endoscope (50) and has one or more grooves (12, 14) formed to extend parallel to each other in a longitudinal direction of the rigid endoscope (50), and a tubular member (40, 42) which is fitted into each groove (12, 14) of the cover member (10) and in which a cleaning fluid to be supplied to an observation window portion of the rigid endoscope (50) flows from a base end side of the rigid endoscope (50). A plurality of discharge ports (22, 24) are formed in the discharge portion (20), and a branch portion (27) which splits the cleaning fluid sent from the tubular member (40, 42) to discharge the cleaning fluid from each discharge port (22, 24) toward the observation window portion is provided.

## Description

### TECHNICAL FIELD

The present invention relates to a cover for covering the outer circumferential surface of a rigid endoscope in which an observation window portion for protecting an objective lens for imaging an affected part is provided at a tip thereof, and an endoscope unit including a rigid endoscope and the cover.

### BACKGROUND ART

In recent years, endoscopes (soft endoscopes), as typified by gastrocameras, have been used for gastric examination and surgery in the medical field. In addition, thoracoscopes are often used in surgery in which the chest is opened, and laparoscopes are often used in surgery in which the abdomen is opened (hereinafter referred to as laparoscopic surgery). Laparoscopic surgery is rapidly gaining popularity due to its low invasiveness and good aesthetic outcome, and the proportion of laparoscopic surgery out of all surgical operations, mainly gastric and colorectal cancer surgeries, is increasing every year. Meanwhile, the above-described thoracoscopes and laparoscopes, etc., are called rigid endoscopes, and, at the tip of such a rigid endoscope, an objective lens for imaging an affected part is provided, and an observation window portion for protecting the objective lens is also provided.

During surgery using a rigid endoscope such as the above-described thoracoscopes and laparoscopes, an observation window portion provided at the tip of the rigid endoscope frequently becomes dirty due to blood, fat, etc., or becomes fogged. Therefore, there is a problem that the surgery has to be temporarily suspended in order to clean or purify the observation window portion, causing a significant decrease in surgical efficiency. In more detail, information necessary for surgery using a rigid endoscope is only image (visual) information obtained by the rigid endoscope, and fogging or dirt on the observation window portion due to blood, fat, etc., during the surgery hinders the safe execution of the surgery. Therefore, the rigid endoscope has to be taken out from the body and cleaned during the surgery, but the time required for this leads to extension of the surgery time, which may be very burdensome to the patient and may lead to unforeseen complications.

In order to solve such problems, as disclosed, for example, in Japanese Patent No. 6242560, etc., a technology is disclosed in which a cover for supplying cleaning water such as a saline solution and gas for blowing off the cleaning water, to the observation window portion of a rigid endoscope, in the body of a patient is attached to the outer circumferential surface of the rigid endoscope.

### SUMMARY OF THE INVENTION

Recently, there is a need for a technology to clean the observation window portion of the rigid endoscope more reliably.

The present invention has been made in view of such circumstances, and an object of the present invention is to provide a cover, for a rigid endoscope, that can be easily attached to or detached from the outer circumferential surface of the rigid endoscope without a cover member being removed therefrom and that allows an observation window portion of the rigid endoscope to be cleaned more reliably, and an endoscope unit.

A cover for a rigid endoscope of the present invention is a cover for covering a portion in a circumferential direction of an outer circumferential surface of a rigid endoscope in which an observation window portion is provided at a tip thereof, the cover including:
a cover member which covers the portion in the circumferential direction of the outer circumferential surface of the rigid endoscope and has one or more grooves formed so as to extend parallel to each other along a longitudinal direction of the rigid endoscope; and
a tubular member which is fitted into each groove of the cover member and in which a cleaning fluid to be supplied to the observation window portion of the rigid endoscope flows from a base end side of the rigid endoscope, wherein
a discharge portion which changes a direction of the cleaning fluid sent from the tubular member and discharges the cleaning fluid toward the observation window portion is formed at a tip of the cover member, and
a plurality of discharge ports are formed in the discharge portion, and a branch portion which splits the cleaning fluid sent from the tubular member to discharge the cleaning fluid from each discharge port toward the observation window portion is provided.

An endoscope unit of the present invention includes:
a rigid endoscope in which an observation window portion is provided at a tip thereof; and
a cover which covers a portion in a circumferential direction of an outer circumferential surface of the rigid endoscope, wherein
the cover has a cover member which covers the portion in the circumferential direction of the outer circumferential surface of the rigid endoscope and has one or more grooves formed so as to extend parallel to each other along a longitudinal direction of the rigid endoscope, and a tubular member which is fitted into each groove of the cover member and in which a cleaning fluid to be supplied to the observation window portion of the rigid endoscope flows from a base end side of the rigid endoscope,
a discharge portion which changes a direction of the cleaning fluid sent from the tubular member and discharges the cleaning fluid toward the observation window portion is formed at a tip of the cover member, and
a plurality of discharge ports are formed in the discharge portion, and a branch portion which splits the cleaning fluid sent from the tubular member to discharge the cleaning fluid from each discharge port toward the observation window portion is provided.

### BRIEF DESCRIPTION OF THE DRAWINGS

FIG. 1 is a front view showing a cover for a rigid endoscope according to an embodiment of the present invention.
FIG. 2 is a top view showing the configuration of the cover shown in FIG. 1, as viewed from the upper side.
FIG. 3 is a side view of the cover shown in FIG. 1, as viewed from the left side.
FIG. 4 is a cross-sectional view of the cover shown in FIG. 2, taken along a line A-A.
FIG. 5 is a cross-sectional view of the cover shown in FIG. 2, taken along a line B-B.
FIG. 6 is a cross-sectional view of the cover shown in FIG. 2, taken along a line C-C.
FIG. 7 is a perspective view showing the configuration of a discharge portion of the cover shown in FIG. 1, etc.
FIG. 8 is a top view showing the configuration of a tip including the discharge portion of the cover shown in FIG. 1, etc.
FIG. 9 is a diagram showing the configuration of the discharge portion of the cover shown in FIG. 8, as viewed from a base end side.
FIG. 10 is a top view showing the configuration of a cover for a rigid endoscope according to another embodiment of the present invention, as viewed from the upper side.
FIG. 11 is a cross-sectional view of the cover shown in FIG. 10, taken along a line D-D.
FIG. 12 is a diagram showing the configuration of a discharge portion of the cover shown in

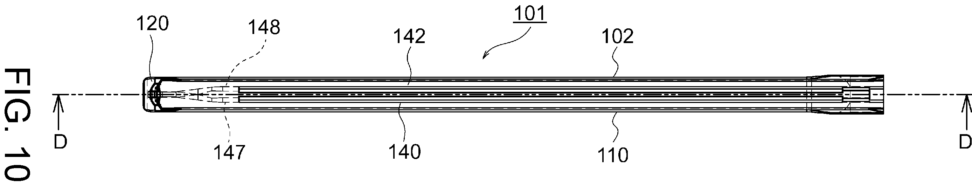

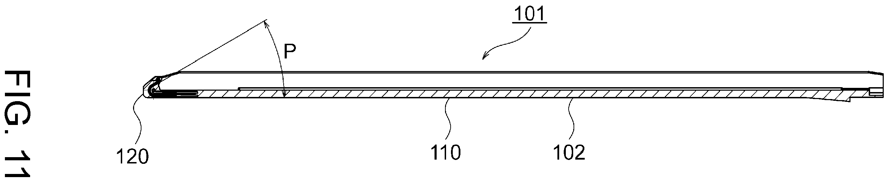

FIG. 10 and FIG. 11, as viewed from a base end side.

### DESCRIPTION OF THE PREFERRED EMBODIMENTS

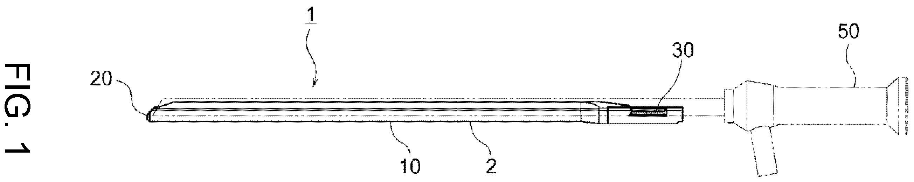

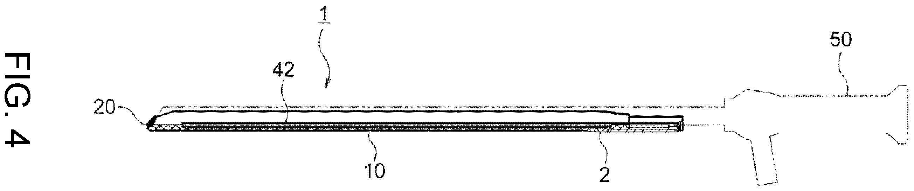

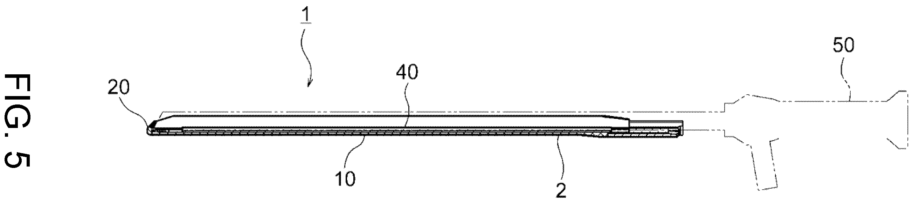

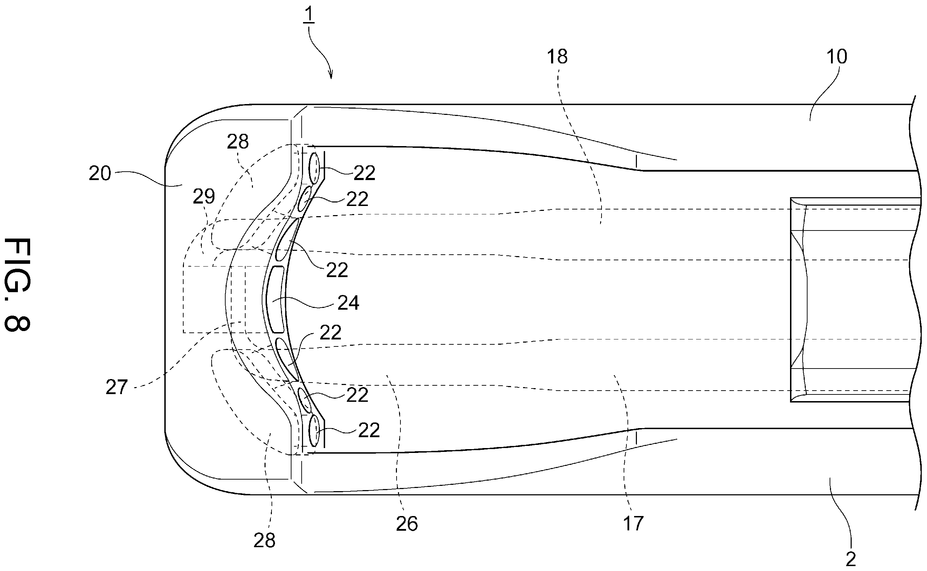

Hereinafter, an embodiment of the present invention will be described with reference to the drawings. FIG. 1 to FIG. 9 show a cover for a rigid endoscope and an endoscope unit according to the present embodiment.

An endoscope unit 1 according to the present embodiment includes a rigid endoscope 50 and a cover 2. Specifically, in the endoscope unit 1 according to the present embodiment, the cover 2 is attached to the outer circumferential surface of the rigid endoscope 50. In FIG. 1 to FIG. 6, the rigid endoscope 50 is shown by an alternate long and two short dashes line, and the cover 2 is shown by a solid line.

First, the rigid endoscope 50 according to the present embodiment will be briefly described. As shown in FIG. 1, etc., the rigid endoscope 50 has an elongated substantially columnar body portion, and an objective lens for imaging an affected part and a transparent or translucent observation window portion such as a glass plate for protecting the objective lens are provided at a tip of the body portion. As shown in FIG. 1, the tip surface (i.e., the surface on the left side in FIG. 1) of the elongated substantially columnar body portion is inclined with respect to a plane orthogonal to the longitudinal direction of the body portion (i.e., the vertical direction in FIG. 1), and the observation window portion is provided on such an inclined tip surface. Thus, the observation window portion is inclined with respect to the plane orthogonal to the longitudinal direction of the elongated substantially columnar body portion. The rigid endoscope according to the present embodiment is not limited to such a rigid endoscope. As another example of the rigid endoscope used in the present embodiment, a rigid endoscope in which an observation member extends along a plane orthogonal to the longitudinal direction of an elongated substantially columnar body portion (i.e., along the vertical direction in FIG. 1) may be used.

Next, the cover 2 for covering a portion in the circumferential direction of the outer circumferential surface of such a rigid endoscope 50 will be described. As shown in FIG. 1 to FIG. 6, etc., the cover 2 according to the present embodiment includes a cover member 10 which covers the portion in the circumferential direction of the outer circumferential surface of the rigid endoscope 50 and has two grooves 12 and 14 formed so as to extend parallel to each other along the longitudinal direction of the rigid endoscope 50, and tubular members 40 and 42 which are fitted into the respective grooves 12 and 14 of the cover member 10 and in which a cleaning fluid to be supplied to the observation window portion of the rigid endoscope 50 flows from the base end side of the rigid endoscope 50.

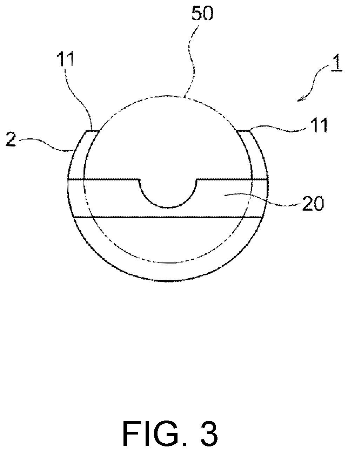

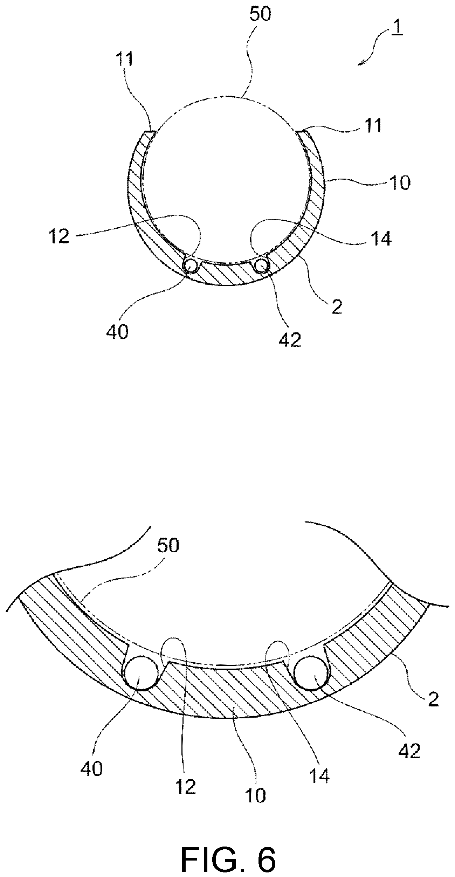

As shown in FIG. 1, etc., the cover member 10 extends over substantially the entire length of the rigid endoscope 50. In addition, as shown in FIG. 3, FIG. 6, etc., the cover member 10 has a substantially C shape when the cover member 10 is viewed along the longitudinal direction, and the rigid endoscope 50 is fitted into the internal space of a C-shaped portion of the cover member 10. Such a cover member 10 is made of plastic such as soft plastic, and the cover member 10 is flexible. As shown in FIG. 3, etc., a pair of capturing portions 11 for capturing the rigid endoscope 50 are formed in the cover member 10. The cover member 10 is attached to the outer circumferential surface of the rigid endoscope 50 by bending the cover member 10. In more detail, when attaching the cover 2 to the rigid endoscope 50, the rigid endoscope 50 is pressed toward the cover member 10 by pressing the outer circumferential surface of the rigid endoscope 50 against the gap of the C-shaped portion of the cover member 10. Accordingly, the cover member 10 is bent, which widens the gap between the pair of capturing portions 11, and the rigid endoscope 50 enters the gap between the pair of capturing portions 11. Therefore, the cover 2 is attached to the rigid endoscope 50 as shown in FIG. 3 and FIG. 6.

Of a pair of the tubular members 40 and 42, the tubular member 40 is configured such that a gas (e.g., carbon dioxide gas) for blowing off a cleaning solution adhering to the observation window portion of the rigid endoscope 50 flows therethrough, and the other tubular member 42 is configured such that a cleaning solution such as a saline solution to be supplied to the observation window portion of the rigid endoscope 50 flows therethrough. A gas supply tube is connected to the tubular member 40, and a gas supply source is connected to the gas supply tube. The gas supplied from the gas supply source to the gas supply tube is sent to the tubular member 40. Thus, when the cover 2 is attached to the rigid endoscope 50, the gas can be supplied from the gas supply tube to the tubular member 40 fitted into the groove 12 of the cover member 10 of the cover 2. In addition, a cleaning solution supply tube is connected to the tubular member 42, and a cleaning solution supply source is connected to the cleaning solution supply tube. The cleaning solution supplied from the cleaning solution supply source to the cleaning solution supply tube is sent to the tubular member 42. Thus, when the cover 2 is attached to the rigid endoscope 50, the cleaning solution can be supplied from the cleaning solution supply tube to the tubular member 42 fitted into the groove 14 of the cover member 10 of the cover 2.

Each of the tubular members 40 and 42 fitted into the respective grooves 12 and 14 of the cover member 10 is made of a flexible rubber or the like. Since each of the tubular members 40 and 42 is flexible, when attaching the cover 2 to the rigid endoscope 50 or detaching the cover 2 from the rigid endoscope 50, the cover member 10 is bent such that the gap between the pair of capturing portions 11 widens, and even if the shapes of the respective grooves 12 and 14 become deformed, the respective tubular members 40 and 42 are also bent in accordance with the deformation of the shapes of the respective grooves 12 and 14. Thus, the respective tubular members 40 and 42 can be inhibited from being damaged or worn, as compared to the case where each of the tubular members 40 and 42 is made of a metal or the like.

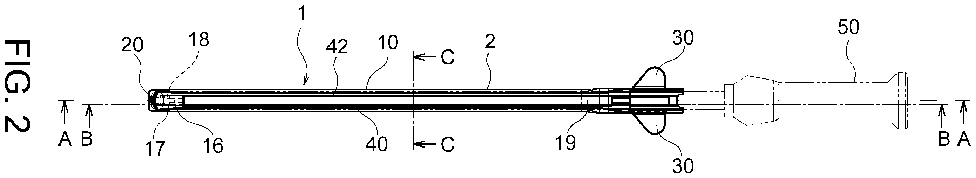

As shown in FIG. 2, etc., retaining portions 16 and 19 which retain the tubular members 40 and 42, which are housed within the respective grooves 12 and 14, in the cover member 10 are provided in the vicinity of the tip and the vicinity the base end of the cover member 10. These retaining portions 16 and 19 can prevent the respective tubular members 40 and 42 from coming out of the respective grooves 12 and 14. In addition, the retaining portion 16 on the side in the vicinity of the tip of the cover member 10 has flow paths 17 and 18 formed therein so as to communicate with the respective tubular members 40 and 42 retained by the retaining portion 16. The tubular member 40 retained by the retaining portion 16 communicates with the flow path 17, and the tubular member 42 retained by the retaining portion 16 communicates with the flow path 18.

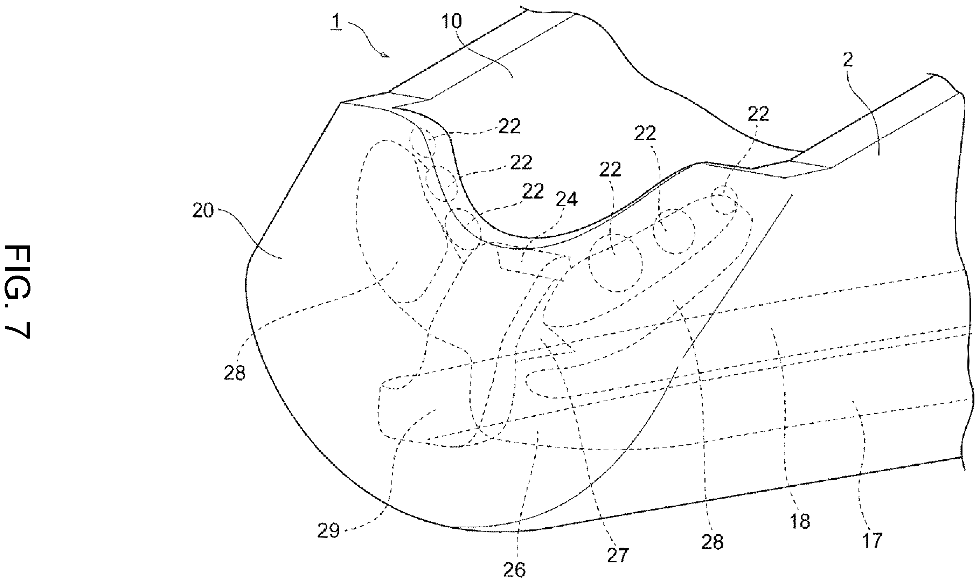

As shown in FIG. 7 to FIG. 9, a discharge portion 20 which changes the directions of the gas and the cleaning solution sent from the respective tubular members 40 and 42 and discharges the gas and the cleaning solution to the observation window portion of the rigid endoscope 50 is formed at the tip of the cover member 10. In addition, a plurality of discharge ports 22 and 24 are formed in the discharge portion 20 such that the gas sent from the tubular member 40 to the discharge portion 20 is discharged from each discharge port 22 and the cleaning solution sent from the tubular member 42 to the discharge portion 20 is discharged from the discharge port 24. Here, each discharge port 22 is formed in the discharge portion 20 such that the gas is discharged from the respective discharge ports 22 in directions different from each other toward the observation window portion of the rigid endoscope 50. The details of the configuration of such a discharge portion 20 will be described below.

Inside the discharge portion 20, a first flow path through which the gas sent from the tubular member 40 passes and a second flow path 29 through which the cleaning solution sent from the tubular member 42 passes are formed. The first flow path includes a flow path 26, a branch portion 27, and a pair of left and right flow paths 28. Here, an upstream end portion of the flow path 26 communicates with the flow path 17 formed inside the retaining portion 16. The branch portion 27 is provided at a downstream end portion of the flow path 26 such that the fluid flowing through the flow path 26 is split from the branch portion 27 into the pair of left and right flow paths 28. Three discharge ports 22 are provided at each of the pair of left and right flow paths 28 such that the fluid split from the branch portion 27 into each flow path 28 is discharged from each discharge port 22. Thus, the gas sent from the tubular member 40 to the discharge portion 20 is discharged from each discharge port 22.

An upstream end portion of the second flow path 29 communicates with the flow path 18 formed inside the retaining portion 16. In addition, the discharge port 24 is provided at a downstream end portion of the second flow path 29. Accordingly, the cleaning solution sent from the tubular member 42 to the discharge portion 20 is discharged from the discharge port 24.

As shown in FIG. 2, etc., a pair of left and right wing members 30 which are grasped by a surgeon when surgery is performed using the rigid endoscope 50 such as a thoracoscope or a laparoscope are provided on the base end side of the cover member 10.

Next, a method for using the endoscope unit 1 having such a configuration will be described. First, when performing surgery in which the endoscope unit 1 is inserted into the body of the patient, the cover 2 is attached to the outer circumferential surface of the rigid endoscope 50. Specifically, the rigid endoscope 50 is pressed toward the cover member 10 by pressing the outer circumferential surface of the rigid endoscope 50 against the gap of the C-shaped portion of the cover member 10. Accordingly, the cover member 10 becomes deformed such that the gap between the pair of capturing portions 11 of the cover member 10 widens, thereby allowing the cover 2 to be attached to the rigid endoscope 50. Accordingly, the endoscope unit 1 in which the cover 2 is attached to the outer circumferential surface of the rigid endoscope 50 is formed. Then, the surgery is performed by inserting the endoscope unit 1 into the body of the patient.

If the observation window portion of the rigid endoscope 50 becomes dirty due to blood, fat, etc., or becomes fogged during the surgery, the observation window portion is cleaned in a state where the endoscope unit 1 is inserted into the body of the patient. In more detail, the cleaning solution is supplied from the cleaning solution supply source to the cleaning solution supply tube connected to the tubular member 42, whereby the cleaning solution flows inside the tubular member 42 in the left direction in FIG. 1. Then, the cleaning solution is sent from the tubular member 42 to the discharge portion 20, and the cleaning solution is discharged from the discharge port 24 through the flow path 18 formed inside the retaining portion 16 and the second flow path 29 formed inside the discharge portion 20. Accordingly, the observation window portion of the rigid endoscope 50 can be cleaned by the cleaning solution discharged from the discharge port 24.

Then, the gas is supplied from the gas supply source to the gas supply tube connected to the tubular member 40, whereby the gas flows inside the tubular member 40 in the left direction in FIG. 1. Then, the gas is sent from the tubular member 40 to the discharge portion 20, and the gas having passed through the flow path 17 formed inside the retaining portion 16 and the flow path 26 formed inside the discharge portion 20 is split at the branch portion 27 to be sent to each of the pair of left and right flow paths 28, whereby the gas is discharged from each discharge port 22. Accordingly, the cleaning solution adhering to the observation window portion of the rigid endoscope 50 can be blown off by the gas discharged from the discharge port 24. At this time, since each discharge port 22 is formed in the discharge portion 20 such that the gas is discharged from the respective discharge ports 22 in directions different from each other toward the observation window portion of the rigid endoscope 50, the cleaning solution adhering to the observation window portion can be blown off more reliably than when the gas is discharged in one direction from the discharge ports 22 toward the observation window portion.

According to the cover 2 of the present embodiment having the above configuration and the endoscope unit 1 including such a cover 2 and the rigid endoscope 50, the cover 2 includes the flexible cover member 10 which covers the portion in the circumferential direction of the outer circumferential surface of the rigid endoscope 50 and has one or more (specifically, two) grooves 12 and 14 formed so as to extend parallel to each other along the longitudinal direction of the rigid endoscope 50, and the tubular members 40 and 42 which are fitted into the grooves 12 and 14 of the cover member 10 and in which the cleaning fluid (specifically, gas or cleaning solution) to be supplied to the observation window portion of the rigid endoscope 50 flows from the base end side of the rigid endoscope 50. In addition, the discharge portion 20 which changes the direction of the cleaning fluid sent from the tubular members 40 and 42 and discharges the cleaning fluid to the observation window portion is formed at the tip of the cover member 10, and the plurality of discharge ports 22 and 24 are formed in the discharge portion 20. Moreover, the branch portion 27 which splits the cleaning fluid sent from the tubular members 40 and 42 to discharge the cleaning fluid from the respective discharge ports 22 and 24 toward the observation window portion is provided. According to such a cover 2, since the cleaning fluid sent from the tubular members 40 and 42 is split at the branch portion 27 and discharged from the respective discharge ports 22 and 24 toward the observation window portion, the cleaning fluid can be discharged from the plurality of discharge ports 22 and 24 toward the observation window portion, whereby the observation window portion can be cleaned reliably.

In the cover 2 of the present embodiment, the respective discharge ports 22 and 24 are formed in the discharge portion 20 such that the cleaning fluid is discharged from the respective discharge ports 22 and 24 in directions different from each other toward the observation window portion. In this case, the cleaning fluid can be discharged from a plurality of directions toward the observation window portion, whereby the observation window portion can be cleaned more reliably.

In the cover 2 of the present embodiment, the tubular members 40 and 42 are flexible. In this case, as described above, when attaching the cover 2 to the rigid endoscope 50 or detaching the cover 2 from the rigid endoscope 50, the cover member 10 is bent, and even if the shapes of the respective grooves 12 and 14 become deformed, the tubular members 40 and 42 are bent in accordance with the deformation of the shapes of the respective grooves 12 and 14. Therefore, the respective tubular members 40 and 42 can be inhibited from being damaged or worn, as compared to the case where each of the tubular members 40 and 42 is made of a metal or the like.

In the cover 2 of the present embodiment, the retaining portions 16 and 19 which retain the tubular members 40 and 42, which are housed inside the grooves 12 and 14, in the cover member 10 are provided in the vicinity of the tip and the vicinity of the base end of the cover member 10. In this case, the tubular members 40 and 42 housed inside the grooves 12 and 14 can be prevented from coming out of the grooves 12 and 14 during surgery.

In the cover 2 of the present embodiment, the two grooves 12 and 14 are formed, and the two tubular members 40 and 42 are provided so as to be fitted into the respective grooves 12 and 14. Then, the tubular member 40 is configured such that the gas flows therethrough as a cleaning fluid, and the other tubular member 42 is configured such that the cleaning solution flows therethrough as a cleaning fluid. In this case, after the cleaning solution is discharged to the observation window portion of the rigid endoscope 50 to clean the observation window portion by supplying the cleaning solution to the tubular member 42, the gas can be discharged to the observation window portion of the rigid endoscope 50 to blow off the cleaning solution adhering to the observation window portion, by supplying the gas to the other tubular member 40.

In the cover 2 of the present embodiment, the flow paths 26 and 28 (first flow path) through which the gas sent from the tubular member 40 passes and the second flow path 29 through which the cleaning solution sent from the tubular member 42 passes are formed inside the discharge portion 20, the first flow path and the second flow path 29 do not communicate with each other, and the gas and the cleaning solution passing through the first flow path and the second flow path 29 are discharged from the different discharge ports 22 and 24, respectively. In this case, since the gas and the cleaning solution can be supplied to the observation window portion of the rigid endoscope 50 through different paths, the effect of cleaning the observation window portion can be further enhanced.

In the cover 2 of the present embodiment, the branch portion 27 is provided in at least one of the first flow path and the second flow path 29. Specifically, the branch portion 27 is provided only in the first flow path, so that the cleaning solution sent from the tubular member 42 to the second flow path 29 is discharged from the discharge port 24 without branching. In this case, the cleaning solution adhering to the observation window portion can be blown off more reliably than when the gas is discharged in one direction from the discharge ports 22 toward the observation window portion.

The cover for a rigid endoscope and the endoscope unit according to the present invention are not limited to the above-described mode, and various modifications can be made.

For example, the mode in which the branch portion 27 is provided only in the first flow path and the cleaning solution sent from the tubular member 42 to the second flow path 29 is discharged from the discharge port 24 without branching has been described above, but the present embodiment is not limited to such a mode. As another mode, a branch portion may also be provided in the second flow path 29, and the cleaning solution sent from the tubular member 42 to the second flow path 29 may branch at this branch portion and be discharged from a plurality of discharge ports in directions different from each other to the observation window portion. In this case, the observation window portion can be cleaned more reliably. As still another mode, a branch portion may be provided in the second flow path 29, the cleaning solution sent from the tubular member 42 to the second flow path 29 may branch at this branch portion and be discharged from a plurality of discharge ports in directions different from each other to the observation window portion, but no branch portion may be provided in the first flow path, and the gas sent from the tubular member 40 to the first flow path may be discharged from one discharge port without branching.

The mode in which the two grooves 12 and 14 are formed in the cover member 10 and the two tubular members 40 and 42 are fitted into the grooves 12 and 14, respectively, has been described above, but the present embodiment is not limited to such a mode. As a cover of another mode, a cover may be used in which one groove is formed in the cover member 10 along the longitudinal direction of the rigid endoscope 50, one tubular member is fitted into this groove, a plurality of discharge ports are formed in a discharge portion, and a branch portion which splits the cleaning fluid sent from the one tubular member to discharge the cleaning fluid from the respective discharge ports toward the observation window portion is provided. In this case, after the cleaning solution is discharged from the respective discharge ports toward the observation window portion by first supplying the cleaning solution to the tubular member, the cleaning solution adhering to the observation window portion is blown off by supplying the gas to the same tubular member to thereby discharge the gas from the respective discharge ports toward the observation window portion. Even with the cover according to such a modification, since the cleaning fluid sent from the tubular member is split at the branch portion and discharged from the respective discharge ports toward the observation window portion, the cleaning fluid can be discharged from the plurality of discharge ports toward the observation window portion, whereby the observation window portion can be cleaned reliably.

In a cover for a rigid endoscope according to still another modification, three or more grooves may be formed in a cover member, and three or more tubular members may be fitted into the respective grooves.

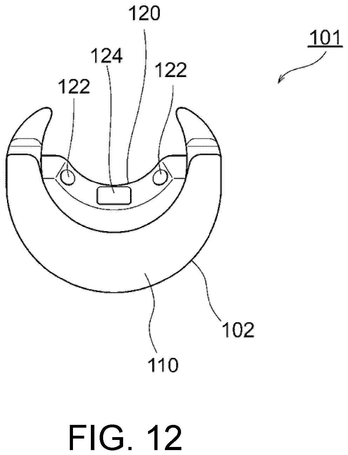

As a cover for a rigid endoscope according to another embodiment of the present invention, one shown in FIG. 10 to FIG. 12 may be used. FIG. 10 is a top view of the cover for a rigid endoscope according the other embodiment of the present invention, as viewed from the upper side, and FIG. 11 is a cross-sectional view of the cover shown in FIG. 10, taken along a line D-D. In addition, FIG. 12 is a diagram showing the configuration of a discharge portion of the cover shown in FIG. 10 and FIG. 11, as viewed from a base end side. In FIG. 10 to FIG. 12, a rigid endoscope is not shown.

A cover 102 of an endoscope unit 101 shown in FIG. 10 to FIG. 12 includes a cover member 110 which covers a portion in the circumferential direction of the outer circumferential surface of a rigid endoscope and has two grooves formed so as to extend parallel to each other along the longitudinal direction of the rigid endoscope, and tubular members 140 and 142 which are fitted into the respective grooves of the cover member 110 and in which a cleaning fluid to be supplied to an observation window portion of the rigid endoscope flows from the base end side of the rigid endoscope. The cover member 110 and the respective tubular members 140 and 142 extend over substantially the entire length of the rigid endoscope. In addition, as shown in FIG. 12, the cover member 110 has a substantially C shape when the cover member 110 is viewed along the longitudinal direction, and the rigid endoscope is fitted into the internal space of a C-shaped portion of the cover member 110. The configuration of the cover member 110 is substantially the same as the configuration of the cover member 10 shown in FIG. 1 to FIG. 9, and thus the description thereof is omitted. Of a pair of the tubular members 140 and 142, the tubular member 140 is configured such that a gas (e.g., carbon dioxide gas) for blowing off a cleaning solution adhering to the observation window portion of the rigid endoscope flows therethrough, and the other tubular member 142 is configured such that a cleaning solution such as a saline solution to be supplied to the observation window portion of the rigid endoscope flows therethrough.

Flow paths 147 and 148 are formed on the side in the vicinity of a tip of the cover member 110 so as to communicate with the respective tubular members 140 and 142, the tubular member 140 communicates with the flow path 147, and the tubular member 142 communicates with the flow path 148.

A discharge portion 120 which changes the directions of the gas and the cleaning solution sent from the respective tubular members 140 and 142 and discharges the gas and the cleaning solution to the observation window portion of the rigid endoscope is formed at the tip of the cover member 110. In addition, a plurality of discharge ports 122 and 124 are formed in the discharge portion 120 such that the gas sent from the tubular member 140 to the discharge portion 120 is discharged from each discharge port 122 and the cleaning solution sent from the tubular member 142 to the discharge portion 120 is discharged from the discharge port 124. Here, each discharge port 122 is formed in the discharge portion 120 such that the gas is discharged from the respective discharge ports 122 in directions different from each other toward the observation window portion of the rigid endoscope.

Inside the discharge portion 120, a first flow path through which the gas sent from the tubular member 140 through the flow path 147 passes and a second flow path through which the cleaning solution sent from the tubular member 142 through the flow path 148 passes are formed. The first flow path is configured such that the gas split into a pair of left and right flow paths to be sent to each flow path is discharged from the pair of left and right discharge ports 122. In addition, an upstream end portion of the second flow path communicates with the flow path 148, and the discharge port 124 is provided at a downstream end portion of the second flow path. Accordingly, the cleaning solution sent from the tubular member 142 to the discharge portion 120 is discharged from the discharge port 124. The first flow path and the second flow path do not communicate with each other inside the discharge portion 120.

Three types of covers were prepared in which angles (reference character P in FIG. 11) of the discharge direction of the gas and the cleaning solution from the respective discharge ports 122 and 124 with respect to the longitudinal direction of the rigid endoscope to which the cover 10 was attached were 15°, 20°, and 30°, respectively.

Next, a method for using the endoscope unit 101 having such a configuration will be described. If the observation window portion of the rigid endoscope to which the cover 102 is attached becomes dirty due to blood, fat, etc., or becomes fogged during surgery, the observation window portion is cleaned in a state where the endoscope unit 101 is inserted into the body of the patient. In more detail, the cleaning solution is supplied from a cleaning solution supply source to a cleaning solution supply tube connected to the tubular member 142, whereby the cleaning solution flows inside the tubular member 142 in the left direction in FIG. 10 and FIG. 11. Then, the cleaning solution is sent from the tubular member 142 to the discharge portion 120, and the cleaning solution is discharged from the discharge port 124 through the flow path 148 and the second flow path formed inside the discharge portion 120. Accordingly, the observation window portion of the rigid endoscope can be cleaned by the cleaning solution discharged from the discharge port 124.

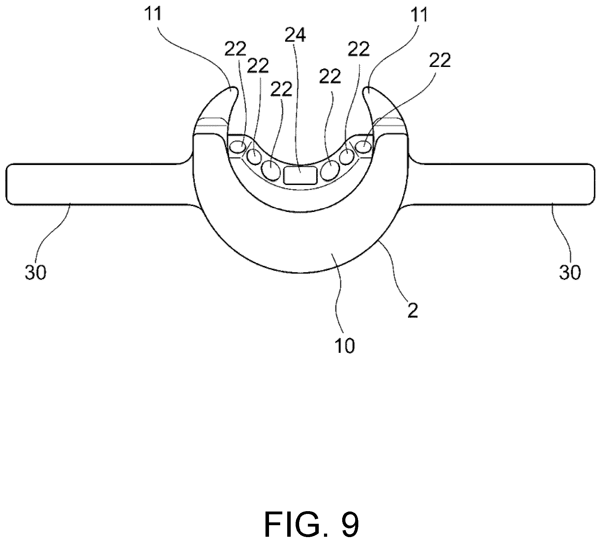

Then, the gas is supplied from a gas supply source to a gas supply tube connected to the tubular member 140, whereby the gas flows inside the tubular member 140 in the left direction in FIG. 9 and FIG. 10. Then, the gas is sent from the tubular member 140 to the discharge portion 120, the gas having passed through the flow path 147 and the first flow path formed inside the discharge portion 120 is split into the pair of left and right flow paths in the middle, and the gas is discharged from each discharge port 122. Accordingly, the cleaning solution adhering to the observation window portion of the rigid endoscope can be blown off by the gas discharged from the discharge port 124. At this time, since each discharge port 122 is formed in the discharge portion 120 such that the gas is discharged from the respective discharge ports 122 in directions different from each other toward the observation window portion of the rigid endoscope, the cleaning solution adhering to the observation window portion can be blown off more reliably than when the gas is discharged in one direction from the discharge ports 122 toward the observation window portion.

Even in such a cover 102 and endoscope unit 101, since the cleaning fluid (specifically, gas) sent from the tubular member 140 is split in the middle and discharged from each discharge port 122 toward the observation window portion, the observation window portion can be cleaned reliably. In more detail, as described above, the three types of covers were prepared in which the angles (reference character P in FIG. 11) of the discharge direction of the gas and the cleaning solution from the respective discharge ports 122 and 124 with respect to the longitudinal direction of the rigid endoscope to which the cover 10 was attached were 15°, 20°, and 30°, respectively, and even at any of the angles, not many water droplets remained on the observation window portion of the rigid endoscope. In particular, when the angle was 30°, almost no water droplets remained on the observation window portion of the rigid endoscope.

As a comparative example, a cover was produced in which a first flow path through which a gas flows and a second flow path through which a cleaning solution flows merge in a discharge portion to form one flow path and the gas or the cleaning solution is discharged toward an observation window portion of a rigid endoscope from a discharge port provided at a downstream end portion of the one flow path. The cleaning of the observation window portion of the rigid endoscope in an endoscope unit in which such a cover was attached to the rigid endoscope, will be described below. By supplying the cleaning solution from a cleaning solution supply source to a cleaning solution supply tube connected to a second tubular member, the cleaning solution was sent from the second tubular member to the discharge portion, and the cleaning solution was discharged from the discharge port through the flow path formed inside the discharge portion, whereby the observation window portion of the rigid endoscope was cleaned by the cleaning solution. Then, by supplying the gas from a gas supply source to a gas supply tube connected to a first tubular member, the gas was sent from the first tubular member to the discharge portion, and the gas was discharged from the discharge port through the flow path formed inside the discharge portion, whereby the cleaning solution adhering to the observation window portion of the rigid endoscope was blown off by the gas. At this time, a problem occurred that many water droplets remained on the observation window portion. In more detail, three types of covers were prepared in which angles of the discharge direction of the gas and the cleaning solution from the discharge port with respect to the longitudinal direction of the rigid endoscope to which the cover was attached were 15°, 20°, and 30°, and at each of the angles, the gas discharged from the discharge port did not hit the observation window portion of the rigid endoscope well, and many water droplets remained on the observation window portion of the rigid endoscope. From such a comparative example, it is found that by branching the gas sent from the first tubular member, inside the discharge portion, and discharging the gas from each discharge port toward the observation window portion, the gas can be discharged from a plurality of discharge ports toward the observation window portion, whereby the cleaning of the observation window portion (specifically, the removal of the cleaning solution) can be performed reliably.

## Claims

1. A cover for a rigid endoscope, the cover being for covering a portion in a circumferential direction of an outer circumferential surface of a rigid endoscope in which an observation window portion is provided at a tip thereof, the cover comprising:
a cover member which covers the portion in the circumferential direction of the outer circumferential surface of the rigid endoscope and has one or more grooves formed so as to extend parallel to each other along a longitudinal direction of the rigid endoscope; and
a tubular member which is fitted into each groove of the cover member and in which a cleaning fluid to be supplied to the observation window portion of the rigid endoscope flows from a base end side of the rigid endoscope, wherein
a discharge portion which changes a direction of the cleaning fluid sent from the tubular member and discharges the cleaning fluid toward the observation window portion is formed at a tip of the cover member, and
a plurality of discharge ports are formed in the discharge portion, and a branch portion which splits the cleaning fluid sent from the tubular member to discharge the cleaning fluid from each discharge port toward the observation window portion is provided.

2. The cover for a rigid endoscope according to claim 1, wherein a pair of capturing portions for capturing the rigid endoscope are formed in the cover member, and the cover member is configured to be bent such that the pair of capturing portions become open to allow attachment or detachment to or from the rigid endoscope.

3. The cover for a rigid endoscope according to claim 1 or 2, wherein the discharge ports are formed in the discharge portion such that the cleaning fluid is discharged from the respective discharge ports in directions different from each other toward the observation window portion.

4. The cover for a rigid endoscope according to any one of claims 1 to 3, wherein the tubular member is flexible.

5. The cover for a rigid endoscope according to any one of claims 1 to 4, wherein retaining portions which retain the tubular member, which is housed inside the groove, in the cover member, are provided in a vicinity of the tip and a vicinity of a base end of the cover member.

6. The cover for a rigid endoscope according to any one of claims 1 to 5, wherein the two grooves are formed, the two tubular members are provided so as to be fitted into the grooves, respectively, one of the tubular members is configured such that a gas flows therethrough as a cleaning fluid, and the other of the tubular members is configured such that a cleaning solution flows therethrough as a cleaning fluid.

7. The cover for a rigid endoscope according to claim 6, wherein a first flow path through which the gas sent from the tubular member passes and a second flow path through which the cleaning solution sent from the tubular member passes are formed inside the discharge portion, the first flow path and the second flow path do not communicate with each other, and the gas and the cleaning solution passing through the first flow path and the second flow path are discharged from the different discharge ports, respectively.

8. The cover for a rigid endoscope according to claim 7, wherein the branch portion is provided in at least one of the first flow path and the second flow path.

9. The cover for a rigid endoscope according to claim 8, wherein the branch portion is provided only in the first flow path, and the cleaning solution sent from the tubular member to the second flow path is discharged from the discharge port without branching.

10. An endoscope unit comprising:
a rigid endoscope in which an observation window portion is provided at a tip thereof; and
a cover which covers a portion in a circumferential direction of an outer circumferential surface of the rigid endoscope, wherein
the cover has a flexible cover member which covers the portion in the circumferential direction of the outer circumferential surface of the rigid endoscope and has one or more grooves formed so as to extend parallel to each other along a longitudinal direction of the rigid endoscope, and a tubular member which is fitted into each groove of the cover member and in which a cleaning fluid to be supplied to the observation window portion of the rigid endoscope flows from a base end side of the rigid endoscope,
a discharge portion which changes a direction of the cleaning fluid sent from the tubular member and discharges the cleaning fluid toward the observation window portion is formed at a tip of the cover member, and
a plurality of discharge ports are formed in the discharge portion, and a branch portion which splits the cleaning fluid sent from the tubular member to discharge the cleaning fluid from each discharge port toward the observation window portion is provided.
